# EUROPEAN PATENT APPLICATION

(11) **EP 4 548 837 A1**
(43) Date of publication of application: **07.05.2025**
(21) Application number: 23207273.6
(22) Date of filing: 01.11.2023
(51) Int. Cl.: A61B 5/02

(54) **TRUNK MODULE FOR AIR SUPPLY ADJUSTMENT**

(71) Applicant: Koninklijke Philips N.V., 5656 AG Eindhoven (NL)
(72) Inventor: MUEHLSTEFF, Jens, Eindhoven (NL); VEENSTRA, Hugo, Eindhoven (NL); HILGERS, Achim Rudolf, 5656AG Eindhoven (NL); DAVIDOIU, Valentina-Geta, Eindhoven (NL); JOYE, Neil Francis, Eindhoven (NL)
(74) Representative: Philips Intellectual Property & Standards

(57) **Abstract**

A trunk module (160A, 160B, 220, 320) includes an air reservoir (120A, 120B); a first pump (121); and an interface (112) configured to connect the trunk module (160A) to an actuator (110). The trunk module is configured to provide supplemental air to the actuator (110) to supplement air output by a second pump (172) to the actuator (110).

## Description

### FIELD OF THE INVENTION

The invention relates to a trunk module for air supply adjustment and a system comprising a trunk module for air supply adjustment.

### BACKGROUND OF THE INVENTION

A pressure range of an air pump inside a patient monitor is designed to cover a range of patients and blood pressure cuffs in order to ensure an accurate non-invasive blood pressure (NIBP) measurement. However, a higher pressure than the air pump can deliver is sometimes needed, or a lower flow rate than the air pump can deliver is sometimes needed. For example, the maximum pressure may be insufficient for blood pressure measurements for obese patients. As another example, the minimum airflow may be too high in a cuff for very small patients or children and new-born patients, resulting in the pressure increasing too quickly. Additionally, an air pump inside a patient monitor is usually not designed for use in tourniquet applications where a high air pressure is needed. As yet another modus which is sometimes not available, a need sometimes exists for an air pump to suck air from a structure attached to a patient, such as to conform a cuff to the patient specific arm geometries.

Examples of varying requirements for air pumps for blood pressure measurements include:
- Deflation-based NIBP with static pressure plateau after a fast cuff inflation - maximum flow and medium pressure range up to 300 mmHg
- Inflation-based NIBP where blood pressure is inferred during the dynamically increased pressure with medium flow rate
- A proprietary actuator cuff inflation with very low flow rates reaching very high cuff pressure up to 600 mmHg

### SUMMARY OF THE INVENTION

The invention is defined by the independent claims. Advantageous embodiments are defined in the dependent claims.

According to an aspect of the present disclosure, a trunk module includes an air reservoir; a first pump; and an interface configured to connect the trunk module to an actuator. The trunk module is configured to provide supplemental air to the actuator to supplement air output by a second pump to the actuator.

According to another aspect of the present disclosure, a system includes a patient monitor; an actuator; and a trunk module. The trunk module includes an air reservoir; a first pump; and an interface configured to connect the trunk module to the actuator. The trunk module is configured to provide supplemental air to the actuator to supplement air output by a second pump to the actuator.

According to another aspect of the present disclosure, a computer implemented method of operation of a trunk module is provided according to claim 14.

According to another aspect of the present disclosure, a computer program is provided according to claim 15.

These and other aspects of the invention will be apparent from and elucidated with reference to the embodiments described hereinafter.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A illustrates a system with a trunk module for air supply adjustment, in accordance with a representative embodiment.
FIG. 1B illustrates another system with a trunk module for air supply adjustment, in accordance with a representative embodiment.
FIG. 2 illustrates another system with a trunk module for air supply adjustment, in accordance with a representative embodiment.
FIG. 3 illustrates another system with a trunk module for air supply adjustment, in accordance with a representative embodiment.
FIG. 4 illustrates a trunk module for air supply adjustment with a reservoir with an adjustable volume, in accordance with a representative embodiment.
FIG. 5 illustrates a trunk module for air supply adjustment valve system, in accordance with a representative embodiment.
FIG. 6 illustrates a method of operation for a trunk module for air supply adjustment, in accordance with another representative embodiment.

### DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to" or "coupled to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, a pump, an air reservoir and a control system are provided in a trunk module to extend the air pressure and/or flow range that can be applied to a flow path towards an attached actuator such as a cuff and/or to suck air from the flow path. The trunk module may be provided physically separate from a patient monitor to extend or to offset the air pressure and/or flow range provided by a pump in the patient monitor. The pump in the trunk module may be provided as a secondary pump to support a higher maximum pressure and/or to reduce a minimum airflow. The air reservoir is an additional air-volume connected in series to the actuator, and may be used to vary the rate of inflation of the actuator. Identification of the actuator and patient monitor may be performed in the trunk module to control the reservoir and one or more pumps in the trunk module to achieve the desired air pressure and/or flow.

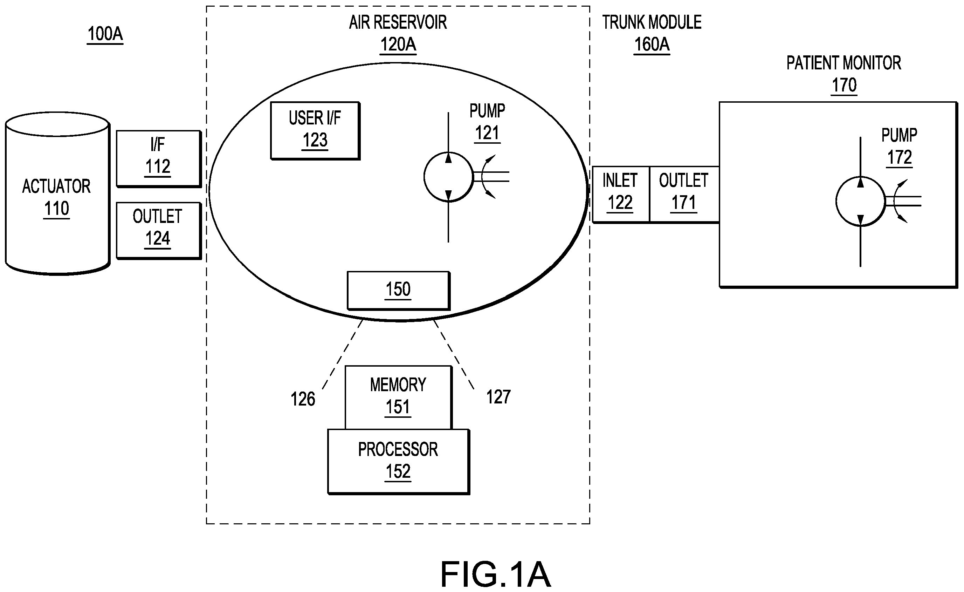

FIG. 1A illustrates a system with a trunk module for air supply adjustment, in accordance with a representative embodiment.

The system 100A in FIG. 1A includes an actuator 110, a trunk module 160A, and a patient monitor 170. The trunk module 160A may be modular and provided in a variety of configurations so as to be attachable and detachable from a variety of types of instances of the actuator 110 and the patient monitor 170. The actuator 110 includes an interface 112 with the trunk module 160A, and specifically with an air reservoir 120A of the trunk module 160A. The trunk module 160A includes the air reservoir 120A, and the air reservoir 120A includes a first pump 121, an inlet 122, an optional user interface 123, an outlet 124, an optional cuff recognition system 126, an optional monitor recognition system 127, and an optional controller 150. The outlet 124 is an air outlet of the trunk module 160A and is connected to the actuator 110. The controller 150 includes a memory 151 that stores instructions and a processor 152 that executes the instructions.

The actuator 110 may comprise a cuff and/or tourniquet. Therefore, the cuff recognition system 126 may be configured to recognize presence and type of a cuff or a tourniquet, and the outlet 124 may supply air to a cuff or a tourniquet.

The user interface 123 may comprise elements such as a touchscreen, buttons, knobs, switches, keys, and may be configured to accept information such as patient information of patient characteristics for the patient using the actuator 110, and/or use of the actuator 110, type of the actuator 110, and/or type of patient monitor 170 connected to the trunk module 160A.

The cuff recognition system 126 may comprise a circuit such as an application-specific integrated circuit (ASIC), or may comprise a software program of instructions stored in the memory 151. The monitor recognition system 127 may comprise a circuit such as an application-specific integrated circuit, or may comprise a software program of instructions stored in the memory.

The patient monitor 170 includes an outlet 171 and a second pump 172. The inlet 122 is an air inlet of the trunk module 160A and the outlet 171 is an air outlet of the patient monitor 170. As shown, the inlet 122 of the trunk module 160A connects to the outlet 171 of the patient monitor 170. The patient monitor 170 may also include a display with one or more input interface(s) such as an interactive touch screen configured to display prompts to users and collect touch input from users.

In FIG. 1A, the first pump 121 and the second pump 172 are separately implemented in the trunk module 160B and the patient monitor 170 respectively. The interface 112 is configured to connect the trunk module 160A to the actuator 110. The trunk module 160A is configured to provide supplemental air to the actuator 110 to supplement air output by the second pump 172 to the actuator 110. The trunk module 160A may also be configured to suck air from the actuator 110 to offset the air output by the second pump 172 to the actuator 110.

The controller 150 is logic circuitry and includes at least the memory 151 that stores instructions and the processor 152 that executes the instructions, and may also include the cuff recognition system 126 and the monitor recognition system 127 as software modules stored in the memory 151. The cuff recognition system 126 is configured to detect a type of the actuator 110 connected to the air reservoir 120A via the interface 112, such as by a signal communicated electronically via the interface 112. The monitor recognition system 127 is configured to detect a type of the patient monitor 170 connected to the trunk module 160A via the outlet 171 and the inlet 122 or another interface, such as by a signal communicated electronically via such another interface.

The controller 150 may perform some of the logical operations described herein directly. The controller 150 may comprise a control system configured to perform logical processing based on digital signals. The controller 150 may comprise a stand-alone device incorporated in the trunk module 160A or in or as another device. The controller 150 may comprise logic circuitry configured to identify a use of the actuator 110, a type of the actuator 110, and/or a type of the patient monitor 170 connected to the trunk module 160A. Provision of supplemental air to the actuator 110 may be varied under control of the controller 150. Also and/or alternatively, offset of air output by the second pump 172 may be controlled by the controller 150 based on identifying at least one of the use of the actuator 110, the type of the actuator 110, and/or the type of patient monitor 170 connected to the trunk module 160A.

The processor 152 may be considered a representative example of a processor of a controller and executes instructions to implement some or all aspects of methods and processes described herein. The processor 152 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 152 is an article of manufacture and/or a machine component. The processor 152 is configured to execute software instructions to perform functions as described in the various embodiments herein. The processor 152 may be a general-purpose processor or may be part of an application specific integrated circuit (ASIC). The processor 152 may be local to the computer system, and/or distributed amongst multiple computer systems or computing devices, or disposed in the `cloud' according to known components and methods.

The processor 152 may also be a microprocessor, a microcomputer, a processor chip, a controller, a microcontroller, a digital signal processor (DSP), a state machine, or a programmable logic device. The processor 152 may also be logic circuitry, including a programmable gate array (PGA), such as a field programmable gate array (FPGA), or another type of circuit that includes discrete gate and/or transistor logic circuitry. The processor 152 may be a central processing unit (CPU), a graphics processing unit (GPU), or both. Additionally, any processor described herein may include multiple processors, parallel processors, or both. Multiple processors may be included in, or coupled to, a single device or multiple devices. The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system. The term computing device should also be interpreted to include a collection or network of computing devices each including a processor or processors. Programs have software instructions performed by one or multiple processors that may be within the same computing device or system.

"Memory" is an example of computer-readable storage media, and should be interpreted as possibly being multiple memories or databases. More generally, the inventive concepts also encompass a computer readable medium that stores instructions that cause a processor to execute the methods described herein. Examples of such media include non-transitory media such as computer memory devices that store information in a format that is readable by a computer or data processing system. More specific examples of non-transitory media include computer disks and non-volatile memories.

The memory or database for instance may be multiple memories or databases local to the computer system 120, and/or distributed amongst multiple computer systems or computing devices, or disposed in the `cloud' according to known components and methods. The memory 151 may comprise one or more memories such as a main memory and a static memory, where memories in the controller 150 communicate with each other and the processor 152 via a bus. The memory 151 may store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 151 is an article of manufacture and/or machine component. The memory 151 is a computer-readable medium from which data and executable software instructions can be read by the processor 152. The memory 151 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memory 151 may be volatile or non-volatile, secure and/or encrypted, unsecure and/or unencrypted. "Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

A control algorithm implemented by a program stored in the memory 151 and executed by the processor 152 of the controller may input parameter information to vary the functionality of the trunk module 160A. The control algorithm may take as input a current application of the trunk module 160A via the user interface 123. Applications of the trunk module 160A may include, for example, non-invasive blood pressure measurements or use of an actuator as a tourniquet, and details of the patient such as age, weight, and size. The control algorithm may also input the type of actuator connected to the trunk module 160A via the cuff recognition system 126. The control algorithm may also input the type of patient monitor connected to the trunk module 160A via the monitor recognition system 127.

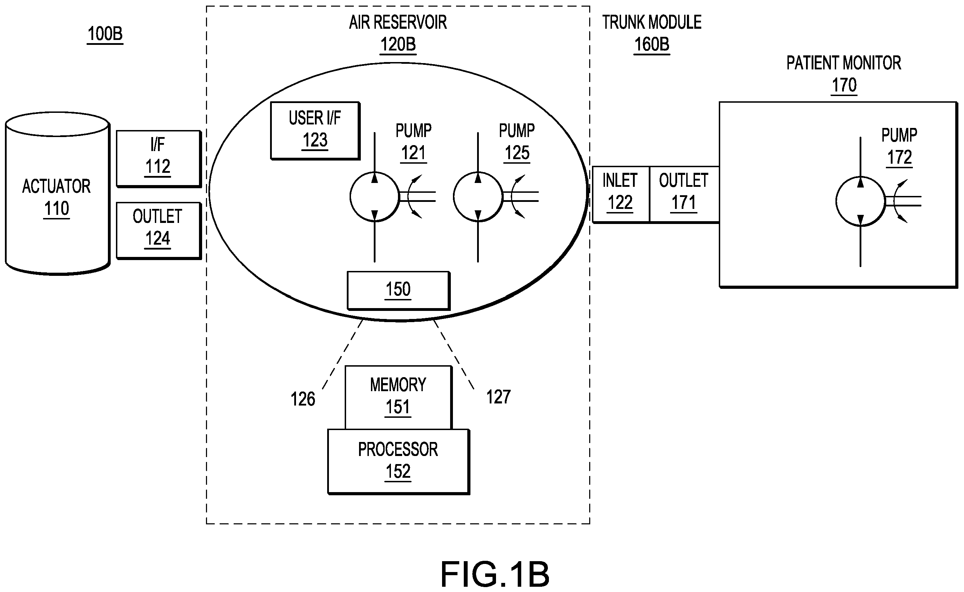

FIG. 1B illustrates another system with a trunk module for air supply adjustment, in accordance with a representative embodiment.

The system 100B in FIG. 1B includes the actuator 110 from FIG. 1A, a trunk module 160B, and the patient monitor 170. The trunk module 160B may be modular and provided in a variety of configurations so as to be attachable and detachable from a variety of types of instances of the actuator 110 and the patient monitor 170. The trunk module 160B includes the air reservoir 120B, a first pump 121, a third pump 125, the inlet 122, the optional user interface 123, the outlet 124, the optional cuff recognition system 126, the optional monitor recognition system 127, and the optional controller 150. The controller 150 includes the memory 151 that stores instructions and the processor 152 that executes the instructions. The patient monitor 170 includes the outlet 171 and the second pump 172.

In FIG. 1B, the first pump 121 and the second pump 172 are separately implemented in the trunk module 160B and the patient monitor 170 respectively. The third pump 125 is aligned in series with the first pump 121 in the trunk module 160B, and may be considered a second pump relative to the first pump 121 in the context of only the trunk module 160B. The interface 112 is configured to connect the trunk module 160B to the actuator 110. The trunk module 160B is configured to provide supplemental air to the actuator 110 to supplement air output by the second pump 172 to the actuator 110. The trunk module 160B may also be configured to suck air from the actuator 110 to offset the air output by the second pump 172 to the actuator 110.

The trunk module 160A, the trunk module 160B and any other trunk module described herein may be configured to extend the air pressure and/or airflow range of an actuator such as a cuff towards lower minimum airflows so as slow the increase of pressure and/or to increase maximum pressure. As such, the trunk module 160A, the trunk module 160B and any other trunk module described herein may be implemented as a general-purpose air pressure/flow module that can be used for non-invasive blood pressure or tourniquet applications. The trunk modules described herein may extend the range of cuffs that can be used in combination with the patient monitor, and may enable support for new cuffs that are enabling new measurements while being backwards compatible. The controller 150 may be embedded inside the trunk modules for control of the second pump 172 in the patient monitor 170 and for the first pump 121 and respective reservoir inside the respective trunk module.

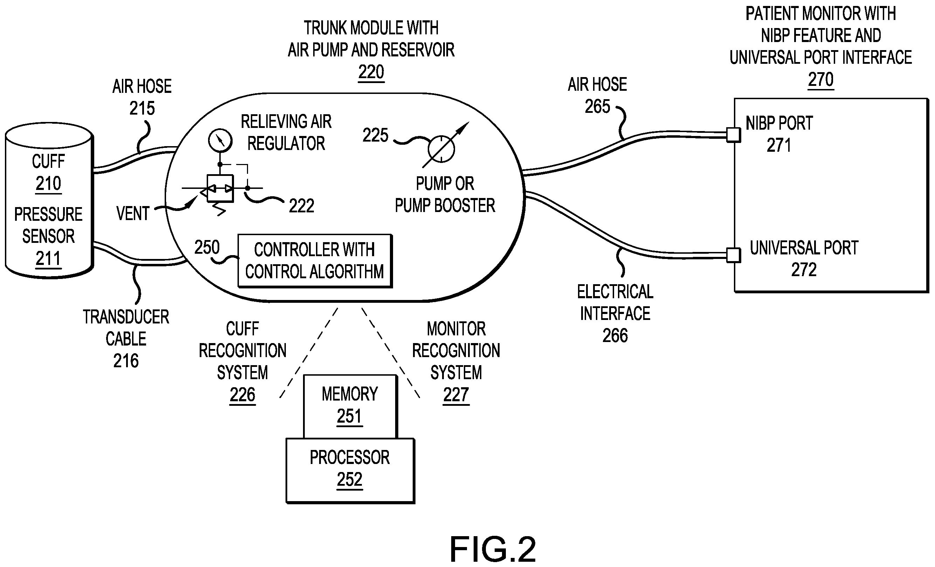

FIG. 2 illustrates another system with a trunk module for air supply adjustment, in accordance with a representative embodiment.

The system in FIG. 2 includes an actuator 210, a trunk module 220, and a patient monitor 270. The trunk module 220 may be modular and provided in a variety of configurations so as to be attachable and detachable from a variety of types of instances of the actuator 210 and the patient monitor 270. The actuator 210 comprises a cuff with a pressure sensor 211. The actuator 210 is connected to the trunk module 220 via an air hose 215 and a transducer cable 216. The trunk module 220 includes an air regulator 222, a first pump 225, a reservoir, an optional controller 250, an optional cuff recognition system 226 and an optional patient monitor recognition system 227. The controller 250 includes a memory 251 that stores instructions and a processor 252 that executes the instruction. The memory 251 stores at least a control algorithm for controlling the trunk module 220. The cuff recognition system 226 and the patient monitor recognition system 227 may comprise circuits or software modules stored in the memory 251 and executed by the processor 252. The patient monitor 270 includes a NIBP port 271 and a universal port 272. The patient monitor 270 is connected to the trunk module 220 by an air hose 265 connected to the NIBP port 271 and an electrical interface 266 connected to the universal port 272. The electrical interface 266 may comprise a power cable configured to provide power from the patient monitor 270 to the trunk module 220.

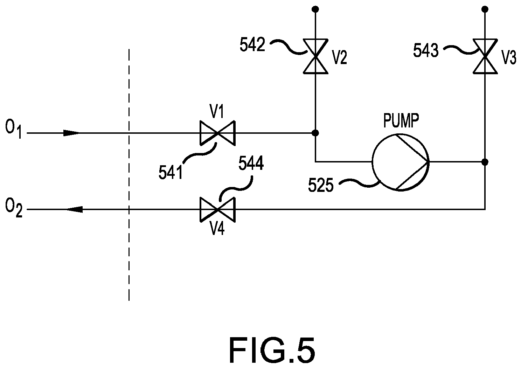

The trunk module 220 is visualized in FIG. 5. The controller 250 controls the air regulator 222 and the first pump 225 inside the trunk module 220, as well as any second pump inside the patient monitor 270. The air regulator 222 may be controlled to regulate an air pressure of the actuator 210 or an air flow provided to the actuator 210. The air regulator 222 may comprise one or more valve(s) configured to control a leakage flow of air from the second pump in the patient monitor 270.

The controller 250 receives inputs as a control signal from the pressure sensor 211 in the actuator 210. Inputs from the pressure sensor may be, for example, input indicating use of the actuator 210, a control signal from the actuator 210 indicating pressure in the actuator 210 such as actual pressure in a cuff. The controller 250 may also receive input from a user via a user interface (not shown) in or on the trunk module 220. Input from a user may include, for example, application of the trunk module 220 and information of the patient. The controller 250 may also receive information from the cuff recognition system 226 and the patient monitor recognition system 227 such as data identifying type of the actuator 210 and the patient monitor 270 connected to the actuator 210 and to the trunk module 220.

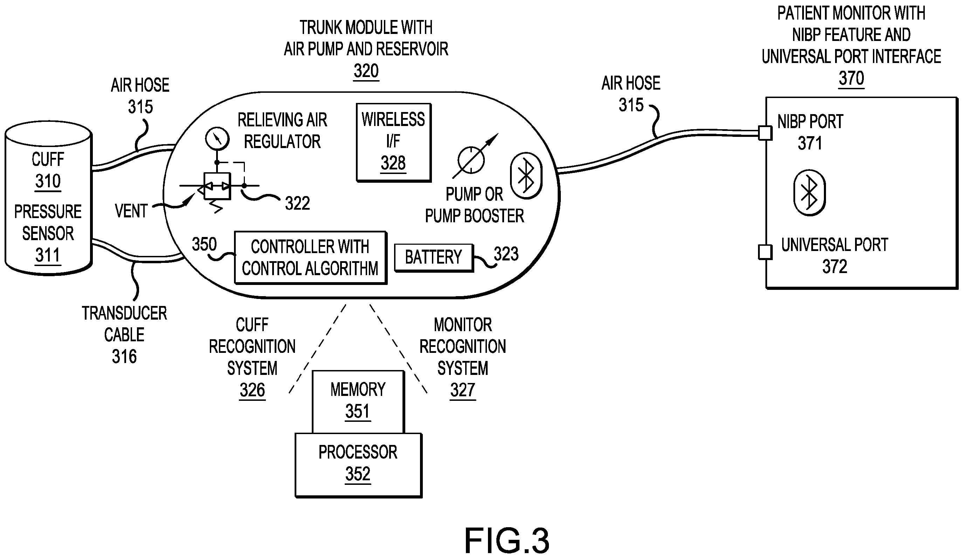

FIG. 3 illustrates another system with a trunk module for air supply adjustment, in accordance with a representative embodiment.

The system in FIG. 3 includes an actuator 310, a trunk module 320, and a patient monitor 370. The trunk module 320 may be modular and provided in a variety of configurations so as to be attachable and detachable from a variety of types of instances of the actuator 310 and the patient monitor 370. The actuator 310 comprises a cuff with a pressure sensor 311. The actuator 310 is connected to the trunk module 320 via an air hose 315 and a transducer cable 316. The trunk module 320 includes an air regulator 322, a first pump 325, a reservoir, a power source (e.g., a battery) 323, a wireless interface 328, an optional controller 350, an optional cuff recognition system 326 and an optional monitor recognition system 327. The controller 350 includes a memory 351 that stores instructions and a processor 352 that executes the instruction. The memory 351 stores at least a control algorithm for controlling the trunk module 320. The cuff recognition system 326 and the monitor recognition system 327 may comprise circuits or software modules stored in the memory 351 and executed by the processor 352. The patient monitor 370 includes a NIBP port 371 and a universal port 372. The patient monitor 370 is connected to the trunk module 320 by an air hose 365 connected to the NIBP port 371. The wireless interface 328 may be configured to enable communications between the trunk module 320 and the patient monitor 370 via the universal port 372. The battery 323 in FIG. 3 is a power source provided to ensure power in the context of the wireless interface 328.

The system in FIG. 3 is an alternative to the system in FIG. 2, and uses a wireless interface between the patient monitor 370 and the trunk module 320. The wireless interface may comprise a Bluetooth connection, for example.

The system in FIG. 1A, FIG. 1B, FIG. 2 and FIG. 3 enable varied solutions for combinations of components in patient monitors and trunk modules. For example, a first requirement for high flow may benefit from extending the maximum flow using a first pump in a trunk module in parallel with a second pump in a patient monitor so as to sum the pump flow capacities towards a specific required value. A second requirement for high pressure may benefit from extending the maximum pressure using the first pump in the trunk module in series with the second pump in the patient module to achieve higher pressure versus maximum pressure per pump. In an alternative solution for the second requirement for high pressure, the reservoir may be pre-pressurized by the second pump in the patient monitor, and the pre-pressurized air in the reservoir may be used to supplement the first pump and the second pump to obtain an even higher pressure. A third requirement for low flow may be benefit from reducing the minimum airflow using a valve in a cable of the trunk module or in the reservoir to create a controlled leakage flow to optimize the required flow. The third requirement may occur when a minimum flow for the second pump in the monitor is too high. A fourth requirement to suck air from an actuator may benefit from a cable for the trunk module sucking air from a device attached to a patient using settings on a valve and the first pump in the trunk module. The fourth requirement may be imposed when a stiff structure at the arm is required. A fifth requirement may be to switch from one mode to another, such as from sucking to pressuring, and this may be addressed using valves and the controllers described herein.

In more detail, the first requirement for extending the maximum air flow may benefit from a quick boost of air in the cuff when the air reservoir implemented in the trunk contains pre-pressurized air. Once that the application is selected (e.g. tourniquet) the air may be released instantaneously in the cuff. The extra air available from the reservoir will significantly decrease the time occlusion of the bleeding limb. This effect of higher air flow in the cuff can also be achieved by connecting the air pumps (patient monitor and trunk) in parallel. Moreover, a special trunk dedicated to external hemorrhage applications can be constructed by implementing multiple pumps in parallel in the trunk, all in parallel with the pump in the patient monitor. The parallel connection where the airflow rate is increased is beneficial for the tourniquet application where a larger cuff (e.g. leg cuff) has to be inflated in a very short time so that the bleeding/hemorrhage is stopped as fast as possible. This dedicated trunk can simplify the workflow of a clinician because the trunk will be automatically identified and no interaction from the clinician is required. The same dedicated trunk can have pumps in parallel and an air reservoir with pressurized air already available.

In more detail, the second requirement for extending the maximum pressure may benefit from the first pump inside the trunk module boosting the maximum output air pressure towards the cuff. This could be realized by using a more powerful pump in the trunk or connecting a pump in the trunk in series to the pump of the patient monitor. A connection of both pumps (trunk and monitor) in parallel may also be advantageous, to increase the maximum airflow towards the cuff. By adding pneumatic switching means, the connection (either in series or parallel) could be adapted according to actual requirements (type of cuff connected or optimal inflation speed/pressure). Finally, if more than one pump in the trunk would be used, and in combination with a variable pneumatic switching means, a very flexible pump behavior could be realized.

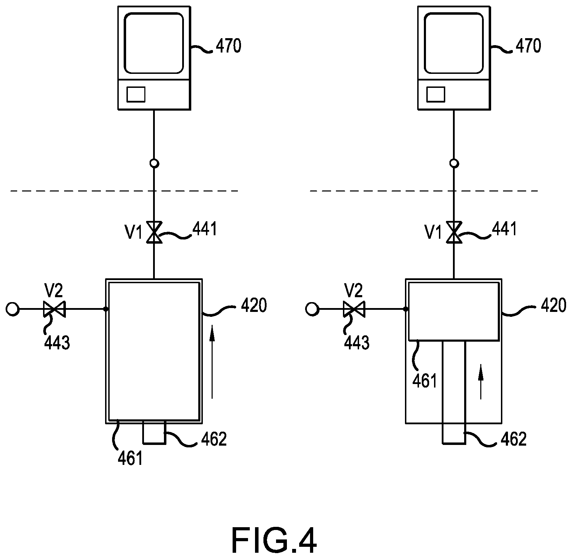

In the variant for the second requirement, a higher maximum pressure than the monitor can deliver may be realized. Assume a maximum pressure is required in a certain use case, but the monitor pump delivers to a given volume only a pressure of PMonitor_max (Volume). To achieve a higher maximum pressure, the trunk module with a reservoir with an adjustable volume may be used. A reservoir in a trunk module with an adjustable volume is shown in and described with respect to FIG. 4 below.

In more detail, the third requirement for reducing minimum airflow to slow the pressure increase may benefit from an air regulator 322 as in the trunk module 320 in FIG. 3. The air regulator 322 can reduce the minimum airflow. The air regulator 322 is controlled by the controller. The reservoir feature can reduce airflow variations which can occur due to non-constant airflow output from the pumps. The generated airflow will be adapted to the current air pressure (as a function of the airflow) and the required pressure increase and final air pressure in the cuff.

In more detail, the fourth requirement and the fifth requirement may be achieved by sucking air from a structure or device at the patient. It can be beneficial to not only pressurize e.g. a cuff attached at the patient, but it is also advantageous to be able to suck air from a means attached to a patient. An example is a means attached to the patient which - by sucking air from the structure - conforms with a patient body specific geometries and in parallel stiffens the structure. The trunk cable includes the appropriate valves, pumps and pump control logic circuitry to implement this modus.

FIG. 4 illustrates a trunk module with a reservoir with an adjustable volume, in accordance with a representative embodiment.

The reservoir 420 in FIG. 4 is provided in a trunk module with a first valve 441, a second valve 443, a piston 461 and a means 462 to move the piston. A monitor 470 is provided with the trunk module in FIG. 4. As shown, the means 462 drives the piston 461 to reduce the volume of the reservoir 420. The means 462 may comprise one or more gears, shafts and other types of mechanical components usable to drive the piston 461. The first valve 441 and the second valve 443 may be controlled to control the volume of the reservoir 420 as the volume is reduced. The procedure for using the adjustable volume in the reservoir 420 in FIG. 4 involves mechanically changing the volume such as by the means 462 at lower speed but higher forces. The means 462 can be connected by the first valve 441 and the second valve 443 to the monitor 470 and by an actuator connected to the patient. The procedure involves a first step of pressurizing, via the second pump in the monitor 470, a reservoir volume Vreservoir to pressure PMonitor_max. PMonitor_max is determined by the second pump in the monitor 470. A second step involves mechanically reducing Vreservoir with the piston 461 and the means 462 to increase to a maximum pressure given by Boyles law for an isothermal process: Pmax = PMonitor_max * Vreservoir / Vreservoir_reduced.

Such a feature can e.g. reduce the effective measurement time of an air cuff, when the air in an air cuff before application to a patients arm is being reduced, since the effective air volume is reduced dramatically. Another positive side effect is, that - because the air volume is minimized and therefore the damping of a larger air volumes - an improved SNR of the pressure oscillation is observed improving the detection and quantification in subsequent signal processing steps.

FIG. 5 illustrates a trunk module for air supply adjustment valve system, in accordance with a representative embodiment.

There may be some benefit to switch to a modus where a second structure is pressurized. The system setup in FIG. 5 realizes an ability to switch pressurization targets. The setup of the trunk module for air supply adjustment valve system in FIG. 5 is configured to realize a system embodiment to suck air from a first socket O1 and create a flow at a second socket 02. The valves V1 and V2, the valves V1 and V3, or the valves V1 and V2 and V3 are configured to connect to supply air pressure or alternatively to connect to a patient monitor.

Associated logic circuitry to steer the valves in order to achieve the switch is shown in and explained below with respect to Table 1 as follows:

**TABLE 1**

| | V1 | V2 | V3 | V4 |
|---|---|---|---|---|
| To Suck Air Via O₁ | Open | Closed | Open | Closed |
| To Create Flow Via O₂ | Closed | Open | Closed | Open |

Table 1 above shows trunk module for air supply adjustment control logic, in accordance with a representative embodiment. The control logic in Table 6 enables a user to steer a particular flow to the first socket O1 and/or the second socket O2 in FIG. 5. As shown, the logic in Table 1 involves setting the first valve V1, the second valve V2, the third valve V3 and the fourth valve V4 to be open or closed, so as to suck such air from a first target via the first socket O1 and/or to create a flow to a second target via the second socket 02.

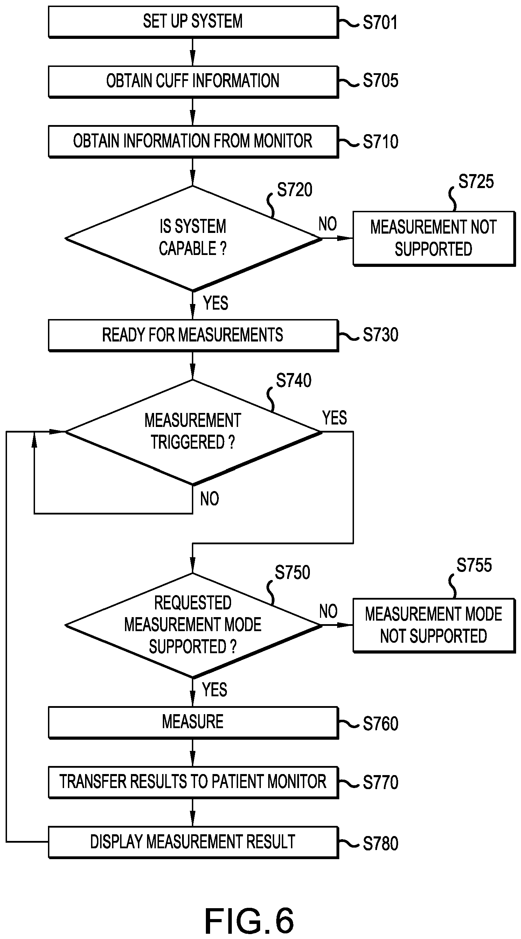

FIG. 6 illustrates a method of operation for a trunk module for air supply adjustment, in accordance with another representative embodiment.

The method of FIG. 6 starts at S701 with setting up a system that includes a trunk module.

At S705, actuator (e.g., cuff) information is obtained, such as by detecting a type of cuff selected by a medical professional to use for monitoring a patient.

At S710, information from a patient monitor is obtained, such as by detecting a type of patient monitor being used in a system with a trunk module.

At S720, a determination is made as to whether the system with the trunk module is capable of performing an intended use. If the system is not capable of performing the intended use (S720 = No), at S725 a determination is made that the measurement is not supported and the process ends. A different cuff and/or patient monitor and/or trunk module may be switched in for use for the intended use.

If the system is capable of performing the intended use (S720 = Yes), at S730 a determination is made that the measurement is supported and the system is ready for measurements.

At S740, a determination is made whether a measurement is triggered. If no measurement is triggered (S740 = No), the determination at S740 is repeated, such as after a predetermined delay. If a measurement is triggered (S740 = Yes), at S750 a determination is made as to whether a requested measurement mode is supported.

If the requested measurement mode is supported (S750 = Yes), at S760 a measurement is taken. If the requested measurement mode is not supported (S750 = No), at S755 a determination is made that the measurement mode is not supported and the process ends. A different cuff and/or patient monitor and/or trunk module may be switched in for use for the intended use.

At S770, results of the measurement at S760 are transferred to the patient monitor.

At S780, the measurement results are displayed, such as on the patient monitor, and the process returns to S740 to repeat the loop for checking whether a measurement is triggered until the next measurement is triggered.

As set forth in the teachings above, a trunk module for air supply adjustment may enable any cuff to be compatible with any patient monitor. The cuff recognition systems and monitor recognition systems described herein provide information on the type of cuff and the type of patient monitor used in a system with a trunk module. Recognition can be implemented in a variety of ways including using quick response (QR) codes attached to the patient monitor and cuff, radio frequency identification (RFID) tags attached to the monitor and cuff, or via the electrical interface for the patient monitor and the pressure sensor of the cuff. In some embodiments, a digital interface may include a unique identification for the patient monitor and for the cuff. In some other embodiments, information of the patient monitor and the cuff may be provided to the controller by the user, such as via a user interface on the trunk module. Cuff recognition may also be implemented by monitoring the pressure sensor output in response to the air pressure applied to the cuff, such as by means of a calibration routine prior to patient measurements. Patient information may include information of patient characteristics such as age, weight, and size, may also be provided as input to the controller, and this information may either be transmitted by the patient monitor to the trunk module via the electrical interface as in FIG. 2, or via a wireless interface such as Bluetooth between the patient monitor and the trunk module as in FIG. 3. In other embodiments, the patient information may be provided by a healthcare professional via the user interface on the trunk module. In addition to the patient details, information such as the facility location (e.g., intensive care unit (ICU) or operating room (OR)) or type of application (NIBP or tourniquet) may also be provided to the controller.

The leakage path provides a means to reduce air pressure/flow. One result of this may be an overall noise reduction. Specifically, there is an acoustic signal with a particular sound level and noise in the pressure signal, which can compromise parameter interference. Since the pumps are most noisy at comparatively low output pressure/flow (low rotational speed), the pumps can be operated at medium air pressure/flow output while reducing output pressure/flow by means of the relieving air regulator.

Accordingly, the trunk module for air supply adjustment enables extension of the air pressure and/or flow range that can be applied towards an attached actuator such as a cuff or other pressure-based actuators. The trunk module for air supply adjustment may also be configured to suck air in a controlled way, using a control system configured to regulate the air pressure and/or flow. The trunk module may be provided physically separate from a patient monitor. The trunk module for air supply adjustment may also be enabled to operate as a standalone device when an air outlet from a patient monitor is missing or not available.

Although the trunk module for air supply adjustment has been described with reference to several exemplary embodiments, it is understood that the words that have been used are words of description and illustration, rather than words of limitation. Although the trunk module for air supply adjustment has been described with reference to particular means, materials and embodiments, the trunk module for air supply adjustment is not intended to be limited to the particulars disclosed; rather the trunk module for air supply adjustment extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

## Claims

1. A trunk module (160A, 160B, 220, 320), comprising:
an air reservoir (120A, 120B);
a first pump (121); and
an interface (112) configured to connect the trunk module to an actuator (110), wherein the trunk module is configured to provide supplemental air to the actuator to supplement air output by a second pump (172) to the actuator.

2. The trunk module of claim 1, wherein the trunk module is configured to suck air from the actuator to offset the air output by the second pump to the actuator.

3. The trunk module of claim 1 or 2, further comprising:
a controller (150) configured to identify at least one of a use of the actuator, a type of the actuator, and a type of patient monitor (170) connected to the trunk module, and to vary provision of supplemental air to the actuator or offset of air output by the second pump (172) based on identifying at least one of the use of the actuator, the type of the actuator, or the type of patient monitor connected to the trunk module.

4. The trunk module of claim 3, wherein the controller comprises a control system configured to regulate an air pressure of the actuator or an air flow provided to the actuator.

5. The trunk module of any of claims 1-4, further comprising:
an air inlet (122) connected to an air outlet (124) of a patient monitor (170), wherein the second pump is provided in the patient monitor; and
an air outlet (124) connected to the actuator.

6. The trunk module of any of claims 1-5, further comprising:
a controller (150) comprising a memory (151) that stores instructions and a processor (152) that executes the instructions, wherein, when executed by the processor, the instructions cause the trunk module to:
receive a control signal from the actuator indicating pressure in the actuator;
receive input indicating use of the actuator;
receive patient information and data identifying type of the actuator and a patient monitor (170) connected to the actuator and to the trunk module; and
control the first pump based on the control signal, the input and the data.

7. The trunk module of any of claims 1-6, further comprising:
a wireless interface (328); and
a power source (323).

8. The trunk module of any of claims 1-7, further comprising:
a valve configured to control a leakage flow of air from the second pump.

9. The trunk module of any of claims 1-8, wherein air in the air reservoir is pre-pressurized and configured to supplement air output to the actuator.

10. The trunk module of any of claims 1-9, further comprising:
the second pump connected in series with the first pump.

11. The trunk module of any of claims 1-9, further comprising:
the second pump connected in parallel with the first pump.

12. The trunk module of any of claims 1-11, further comprising:
a user interface (123) configured to accept information of patient characteristics, for the patient using the actuator.

13. A system (100A, 100B), comprising:
a patient monitor (170, 270, 370);
an actuator (110, 210, 310); and
a trunk module according to any of claims 1-12.

14. A computer implemented method of operation of a trunk module, the method comprising:
setting up a system according to claim 13 (S701);
obtaining actuator information (S705);
obtaining patient monitor information (S710);
determining if the system with the trunk module is capable of performing an intended use (S720);
determining if a measurement is triggered (S740);
determining if a measurement mode is supported (S750);
taking a measurement (S760), based on the obtained actuator information, the patient monitor information, and based on whether the system with the trunk module is capable of performing the intended use, whether the measurement is triggered and whether the measurement mode is supported.

15. A computer program comprising instructions to cause the system of claim 13 to execute the steps of the method of claim 14.
